# EUROPEAN PATENT SPECIFICATION

(11) **EP 0 717 736 B1**
(45) Date of publication and mention of the grant of the patent: **19.05.2004**
(21) Application number: 94926731.4
(22) Date of filing: 12.09.1994
(51) Int. Cl.: C07D 255/02, C07D 487/04, C07F 9/60, C07F 9/6509, C07D 213/40

(54) **PHOSPHORUS DERIVATIVES TO TREAT AIDS**
PHOSPHORDERIVATE ZUR BEHANDLUNG VON AIDS
DERIVES PHOSPHORES POUR TRAITER LE SIDA

(30) Priority: 10.09.1993 AU PM116193; 24.06.1994 AU PM644694
(43) Date of publication of application: 26.06.1996
(73) Proprietor: NARHEX LIMITED, Hong Kong (HK)
(72) Inventor: GROBELNY, Damian, Watsonia North, VIC 3087 (AU)
(74) Representative: Ablewhite, Alan James
(86) International application number: PCT/AU1994/000538
(87) International publication number: WO 1995/007269

(56) References cited:
- EP-A- 0 432 595
- EP-A- 0 519 433
- EP-A- 0 528 242
- EP-A- 0 574 135
- WO-A-90/09191
- WO-A-91/08221
- WO-A-91/10442
- WO-A-92/15319
- WO-A-92/21696
- AU-A- 1 081 292
- AU-A- 1 835 592
- AU-A- 1 937 392
- AU-A- 2 194 492
- AU-A- 2 288 992
- AU-A- 2 412 992
- AU-A- 2 469 092
- AU-A- 2 642 492
- AU-A- 3 162 893
- AU-A- 3 516 593
- AU-A- 3 562 193
- AU-A- 3 570 089
- AU-A- 3 716 093
- AU-A- 3 739 193
- AU-A- 3 880 893
- AU-A- 4 123 093
- AU-A- 4 165 993
- AU-A- 4 230 889
- AU-A- 4 493 093
- AU-A- 4 566 589
- AU-A- 4 611 589
- AU-A- 4 907 293
- AU-A- 5 371 690
- AU-A- 6 207 094
- AU-A- 6 322 190
- AU-A- 6 633 490
- AU-A- 7 131 991
- AU-A- 7 132 091
- AU-A- 7 132 391
- AU-A- 7 732 691
- AU-A- 8 191 091
- AU-A- 8 205 491
- AU-A- 8 231 391
- AU-A- 8 320 691
- AU-A- 8 587 791
- AU-A- 8 730 991
- AU-A- 8 740 991
- AU-A- 8 759 491
- AU-A- 8 890 091
- AU-A- 8 994 191
- AU-A- 9 053 191
- AU-A- 9 085 191
- AU-A- 9 092 591
- AU-A- 9 122 391
- AU-A- 9 125 191
- AU-A- 9 133 291
- AU-A- 9 179 091
- US-A- 5 116 835
- US-A- 5 126 326
- US-A- 5 132 400
- US-A- 5 145 951

## Description

### TECHNICAL FIELD

The invention relates to certain hydrocarbon derivatives bearing polar substituents and their use in the inhibition of retroviral proteases, for example in the treatment of HIV viral infections such as acquired immunodeficiency syndrome (AIDS).

### BACKGROUND ART

Human immunodeficiency virus (HIV) is a pathogenic retrovirus causing AIDS and its related disorders. The development of antiviral chemotherapy against AIDS has been the subject of an intense research effort since the discovery of HIV. (For a recent review on molecular targets for AIDS therapy see Mitsua et al, *Science,* 1990, pp 1533-1544). The HIV Proteases (HIV PR), and aspartyl proteases, were first suggested as a potential target for AIDS therapy by Kramer et al. (*Science* **231,** 1580 (1986)). Since that time the potential usefulness of HIV PR inhibitors as effective agents in treatment of AIDS has been widely recognized (for a review of the HIV PR as a therapeutic target see Tomaselli et al. *Chimica Oggi*, May 1991, pp 6-27 and Huff J.R., *J. Med. Chem.* **34,** 2314-2327 (1991)). Of the classical transition state mimics for aspartyl proteases, the hydroxyethylene, dihydroxyethylene, hydroxyethylamine and phosphinic acid isosteres appear to provide the greatest affinity for HIV PR. Many inhibitors of HIV PR have been shown to have an antiviral activity at concentrations in the nanomolar range in the different cell systems and are described as such in the patent literature.

### OBJECTS OF THE INVENTION

It is an object of the present invention to provide compounds useful as retroviral protease inhibitors. It is another object of the present invention to provide pharmaceutical compositions comprising such compounds and the use of such compounds for the manufacture of medicaments.

### SUMMARY OF THE INVENTION

The invention provides compounds which are useful as inhibitors of retroviral proteases, particularly aspartyl proteases and more particularly HIV proteases, and which are effective in treating conditions characterized by unwanted activity of these enzymes, in particular acquired immune deficiency syndrome.

In the following description of the invention, the teaching of each of the publications mentioned is incorporated herein by reference.

According to a first embodiment of this invention there is provided a compound selected from the group consisting of:
cis-1,6-3-t-Butoxycarbonyl-4-[(2S, 3S)-2-phosphonooxy-3-(N-quinaldyl-L-asparaginyl)amino-4-phenylbutyl]-3,4-diaza-bicyclo[4.4.0]decane;
cis-1,6-3-t-Butoxycarbonyl-4-[(2S, 3S)-2-phosphitooxy-3-(N-quinaldyl-L-asparaginyl)amino-4-phenylbutyl]-3,4-diaza-bicyclo[4.4.0]decane;
t-Butyl 3-isopropyl-3-[(2S, 3S)-2-phosphonooxy-3-(N-quinaldyl-L-asparaginyl)amino-4-phenylbutylcarbazate; and
t-Butyl 3-isopropyl-3-[(2S, 3S)-2-phosphitooxy-3-(N-quinaldyl-L-asparaginyl)amino-4-phenylbutylcarbazate.

According to a second embodiment of this invention there is provided a pharmaceutical composition comprising an effective amount of a compound of the first embodiment together with at least one pharmaceutically acceptable carrier, diluent, excipient and/or adjuvant.

The composition of the second embodiment may be for oral administration.

According to a third embodiment of this invention there is provided the use of a compound according to the first embodiment for the manufacture of a medicament for inhibiting a retroviral protease in a mammal in need of such retroviral protease inhibition.

The retroviral protease is an HIV protease. The medicament may be for oral administration.

According to a fourth embodiment of this invention there is provided the use of a compound according to the first embodiment for the manufacture of a medicament for the treatment or prophylaxis of acquired immune deficiency syndrome in a mammal in need of such treatment.

Suitable pharmaceutically acceptable salts of the compound of the compounds of the first embodiment are, where the compound contains a basic nitrogen atom, acid addition salts of pharmaceutically acceptable inorganic acids such as hydrochloric, sulfuric, phosphoric, nitric, carbonic, boric, sulfamic, hydrobromic or hydriodic, or with pharmaceutically acceptable organic acids such as acetic, propionic, butyric, tartaric, maleic, hydroxymaleic, methylmaleic, fumaric, malic, citric, lactic, mucic, gluconic, glucoheptonic, glucaric, glucuronic, lactobionic, benzoic, naphthoic, succinic, oxalic, phenylacetic, methanesulphonic, ethanesulfonic, 2-hydroxyethanesulfonic, ethane-1,2-disulfonic, laurylsulfonic, toluenesulphonic, benzenesulphonic, naphthalene-2-sulfonic, salicylic, 4-aminosalicylic, sulphanilic, aspartic, glutamic, edetic, stearic, palmitic, oleic, lauric, pantothenic, tannic, ascorbic, valeric, glycolic, cinnamic, mandelic, 2-phenoxybenzoic, 2-acetoxybenzoic, embonic, nicotinic, isonicotinic, N-cyclohexylsulfamic or other acidic organic compounds, such as 2- or 3-phosphoglycerate and glucose-6-phosphate. Where the compound contains an acid group, suitable pharmaceutically acceptable salts of the compound are addition salts of pharmaceutically acceptable bases such as lithium, sodium, potassium, ammonium, magnesium, calcium and zinc salts, or salts formed with organic amines such as methylamine, dimethylamine, trimethylamine, ethylamine, diethylamine, triethylamine, N-methyl-N-ethylamine, mono-, bis- or tris-(2-hydroxyethyl)amine, 2-hydroxy-tert-butylamine, tris(hydroxymethyl)methylamine, N,N-dimethyl-N-(2-hydroxyethyl)-amine, tri-(2-hydroxyethyl)-amine, N-methyl-D-glucamine, or tributylamine. Compounds of the first embodiment having acid and basic groups can also form internal salts. Other suitable salts are described, for example, in S. M. Berge, *et al*., "Pharmaceutical Salts" J. Pharm. Sci., **66** 1-19 (1977) which is incorporated herein by reference.

The expression "prodrug" as used herein refers to a pharmaceutically acceptable derivative of a compound of the first embodiment which is transformed into a compound of of the first embodiment after administration of the prodrug to a living animal or human, and which has enhanced stability, delivery characteristics and/or therapeutic value compared to the compound of of the first embodiment from which it derives.

The compounds the first embodiment can exist in optically isomeric forms Disclosed herein are all these forms in all proportions including all diastereoisomers and mixtures thereof and all enantiomers, mixtures of enantiomers and racemic mixtures. Where a double bond occurs in the compound of the invention, the double bond may be present in the cis- (Z) or trans- (E) configuration. It will be understood that only compounds of formula (I) with combinations of substituents or functional groups which give rise to stable compounds, are within the scope of the present invention.

The compounds of the first emodiment general formula (I) may be prepared by methods known generally in the art. Suitable methods for the synthesis of compounds of formula (I) and intermediates thereof are described, for example, in Houben-Weyl, *Methoden der Organischen Chemie;* J. March, *Advanced Organic Chemistry*, 3rd Edition (John Wiley & Sons, New York, 1985); D. C. Liotta and M. Volmer, eds, *Organic Syntheses Reaction Guide* (John Wiley & Sons, Inc., New York, 1991); R. C. Larock, *Comprehensive Organic Transformations* (VCH, New York, 1989), H. O. House, *Modern Synthetic Reactions* 2nd Edition (W. A. Benjamin, Inc., Menlo Park, 1972); N. S. Simpkins, ed. *100 Modern Reagents* (The Royal Society of Chemistry, London, 1989); A. H. Haines *Methods for the Oxidation of Organic Compounds* (Academic Press, London, 1988) and B. J. Wakefield *Organolithium Methods* (Academic Press, London, 1988).

Compounds in accordance with the present invention which do not include a solubilising group typically exhibit low to very low water solubility. Inhibitors of HIV proteases which have hitherto been described, and many other pharmaceutically or veterinarily active substances also typically exhibit low to very low water solubility. This property tends to cause the bioavailability of such substances to be relatively low. There is thus a need for a HIV protease inhibitor having enhanced water solubility. Surprisingly, it has been found that the inclusion of a solubilising group as defined herein in a substance having low to very low water solubility results in enhancement of the water solubility of the substance. Thus, substances in accordance with the invention which include a solubilising group exhibit superior bioavailability, including superior oral bioavailability, compared to compounds in accordance with the invention which do not include a solubilising group.

Generally, a compound disclosed herein includes at least one solubilising group selected from

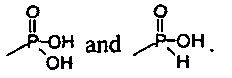

Typically, a solubilising group is introduced into the molecule as the last stage of its synthesis. For example, a solubilising group P(O)(OH)₂ may be introduced to a free amino, hydroxy or mercapto group by reaction of the amino, hydroxy or mercapto group with dimethyl chlorophosphate, followed by mild hydrolysis to remove the methyl ester groups. Alternatively, a solubilising group P(O)(OH)₂ may be introduced to a free hydroxy group by reaction with phosphorous acid and mercuric salts in the presence of a tertiary amine, as described by Obata and Mukaiyama in *J. Org. Chem.,* 32, 1063 (1967). As a further alternative, an amino, hydroxyl or mercapto group may be reacted with phosphorous acid preferably in the presence of a coupling agent such as dicyclohexylcarbodiimide and pyridine to yield a molecule possessing the solubilising group -OP(O)(OH)H. Optionally, this group may be oxidised to the corresponding phosphate derivative, for example using bis(trimethylsilyl) peroxide. A further process for the introduction of a group -P(O)(OH)₂ is described in Australian patent application no. 54311/86, and involves the reaction of an amino, hydroxy or mercapto group with certain diesters of amides of phosphorus acid, followed by oxidation and hydrolysis of the resulting intermediate compounds.

Other methods for the preparation of compounds of the first embodiment referred to herein are disclosed in US Patent Nos. 5,116,835, 5,126,326; 5,132,400; 5,145,957; 5,198,426; 5,212,157; 5,215,968; 5,212,667; 5,294,720; and 5,296,604; International Patent Application Nos. 91/08221; 91/10442; 92/151319 and 92/21696; European Patent Application Nos. 0528242; 0519433 and 0432595 and Australian Patent Application Nos. 35700/89; 53716/90; 63221/90; 71319/91; 71320/91; 71323/91; 82313/91; 83206/91; 87594/91; 90531/91; 90851/94; 90925/91; 91251/91; 91332/91; 18355/92; 26424/92; 37160/93; 38808/93 and 44930/93, the disclosures of each of which are incorporated herein by reference.

Further disclosed herein is a method for inhibiting retroviral proteases in a mammal in need of such inhibition, comprising administering to the mammal an effective amount of a compound of the first embodiment or of a composition of the second embodiment. In one form disclosed herein, there is disclosed a method for the treatment or prophylaxis of HIV viral infections such as AIDS.

For inhibiting retroviral proteases or the treatment of HIV viral infections, a composition of the second embodiment may be administered orally, topically, parenterally, e.g. by injection and by intra-arterial infusion, rectally or by inhalation spray.

For oral administration, the pharmaceutical composition may be in the form of tablets, lozenges, pills, troches, capsules, elixirs, powders, including lyophilised powders, solutions, granules, suspensions, emulsions, syrups and tinctures. Slow-release, or delayed-release, forms may also be prepared, for example in the form of coated particles, multi-layer tablets or microgranules.

Solid forms for oral administration may contain pharmaceutically acceptable binders, sweeteners, disintegrating agents, diluents, flavourings, coating agents, preservatives, lubricants and/or time delay agents. Suitable binders include gum acacia, gelatin, corn starch, gum tragacanth, sodium alginate, carboxymethylcellulose or polyethylene glycol. Suitable sweeteners include sucrose, lactose, glucose, aspartame or saccharine. Suitable disintegrating agents include corn starch, methylcellulose, polyvinylpyrrolidone, xanthan gum, bentonite, alginic acid or agar. Suitable diluents include lactose, sorbitol, mannitol, dextrose, kaolin, cellulose, calcium carbonate, calcium silicate or dicalcium phosphate. Suitable flavouring agents include peppermint oil, oil of wintergreen, cherry, orange or raspberry flavouring. Suitable coating agents include polymers or copolymers of acrylic acid and/or methacrylic acid and/or their esters, waxes, fatty alcohols, zein, shellac or gluten. Suitable preservatives include sodium benzoate, vitamin E, alpha-tocopherol, ascorbic acid, methyl paraben, propyl paraben or sodium bisulphite. Suitable lubricants include magnesium stearate, stearic acid, sodium oleate, sodium chloride or talc. Suitable time delay agents include glyceryl monostearate or glyceryl distearate.

Liquid forms for oral administration may contain, in addition to the above agents, a liquid carrier. Suitable liquid carriers include water, oils such as olive oil, peanut oil, sesame oil, sunflower oil, safflower oil, arachis oil, coconut oil, liquid paraffin, ethylene glycol, propylene glycol, polyethylene glycol, ethanol, propanol, isopropanol, glycerol, fatty alcohols, triglycerides or mixtures thereof.

Suspensions for oral administration may further comprise dispersing agents and/or suspending agents. Suitable suspending agents include sodium carboxymethylcellulose, methylcellulose, hydroxypropylmethylcellulose, polyvinylpyrrolidone, sodium alginate or cetyl alcohol. Suitable dispersing agents include lecithin, polyoxyethylene esters of fatty acids such as stearic acid, polyoxyethylene sorbitol mono- or di-oleate, -stearate or -laurate, polyoxyethylene sorbitan mono- or di-oleate, -stearate or -laurate and the like.

The emulsions for oral administration may further comprise one or more emulsifying agents. Suitable emulsifying agents include dispersing agents as exemplified above or natural gums such as gum acacia or gum tragacanth.

For topical administration, the pharmaceutical composition may be in the form of a cream, ointment, gel, jelly, tincture, suspension or emulsion. The pharmaceutical composition may contain pharmaceutically acceptable binders, diluents, disintegrating agents, preservatives, lubricants, dispersing agents, suspending agents and/or emulsifying agents as exemplified above.

For parenteral administration, the compound of of the first embodiment or its salt may be prepared in sterile aqueous or oleaginous solution or suspension. Suitable non-toxic parenterally acceptable diluents or solvents include water, Ringer's solution, isotonic salt solution, 5% dextrose in water, buffered sodium or ammonium acetate solution, 1,3-butanediol, ethanol, propylene glycol or polyethylene glycols in mixtures with water. Aqueous solutions or suspensions may further comprise one or more buffering agents. Suitable buffering agents include sodium acetate, sodium citrate, sodium borate or sodium tartrate, for example. Aqueous solutions for parenteral administration are also suitable for administration orally or by inhalation.

For rectal administration, the compound of formula I is suitably administered in the form of an enema or suppository. A suitable suppository may be prepared by mixing the active substance with a non-irritating excipient which is solid at ordinary temperatures but which will melt in the rectum. Suitable such materials are cocoa butter, waxes, fats, glycerol, gelatin and polyethylene glycols. Suitable enemas may comprise agents as exemplified above with reference to forms for topical administration.

Suitably, an inhalation spray comprising a compound of the first embodiment will be in the form of a solution, suspension or emulsion as exemplified above. The inhalation spray composition may further comprise an inhalable propellant of low toxicity. Suitable propellants include carbon dioxide or nitrous oxide.

The dosage form of the compound of the first embodiment will comprise from 0.01% to 99% by weight of the active substance. Usually, dosage forms according to the invention will comprise from 0.1% to about 10% by weight of the active substance.

The compound of the first embodiment may be administered together or sequentially with one or more other active substances known or believed to have anti-viral activity. Examples of such other active substances include AZT, acyclovir, ddC, ddA, trisodium phosphonoformate, castanospermine, rifabutin, ribaviran, bropirimine, phosphonothioate oligodeoxynucleotides, dextran sulfate, α-interferon and ampligen.

### BRIEF DESCRIPTION OF THE DRAWINGS

Figure 1 is a graph showing the transformation of the compound of Example 5 ("Prodrug") into the compound of Example 20 of International Patent Application No. PCT/AU93/00103 ("Drug") in rabbit's blood *in vitro.*
Figures 2 and 3 are graphs showing the transformation of Prodrug into Drug *in vivo* following intravenous and intramuscular (respectively) administration to a rabbit.

### BEST MODE OF CARRYING OUT THE INVENTION

Compositions of the second embodiment may be prepared by means known in the art for the preparation of pharmaceutical compositions including blending, grinding, homogenising, suspending, dissolving, emulsifying, dispersing and mixing of the compound of formula (I) together with the selected excipient(s), carrier(s), adjuvant(s) and/or diluent(s).

In the method for the treatment of HIV viral infections disclosed herein, a compound of the first embodiment will usually be administered orally or by injection. A suitable treatment may consist of the administration of a single dose or multiple doses of the compound of the first embodiment or of a composition of the second embodiment. Usually, the treatment will consist of administering from one to five doses daily of the compound of the first embodiment for a period of from one day to several years, up to the lifetime of the patient. Most usually, the treatment will consist of the administration of the compound of the first embodiment or for a period of from one day to one year.

The administered dosage of the compound of the first embodiment can vary and depends on several factors, such as the condition of the patient. Dosages will range from 0.01mg to 200 mg per kg. Usually, the dose of the active substance will be from 0.01mg to 25 mg per kg of body weight.

Examples of dosage forms disclosed herein are as follows:

| 1. | Tablet | |
|---|---|---|
| | Compound of 1^{st} embodiment | 0.01 to 20 mg, generally 0.1 to 10mg |
| | Starch | 10 to 20 mg |
| | Lactose | 100 to 250 mg |
| | Gelatin | 0 to 5 mg |
| | Magnesium stearate | 0 to 5 mg |
| | | |

| 2. | Capsule | |
|---|---|---|
| | Compound of 1^{st} embodiment | 0.01 to 20 mg, generally 0.1 to 10mg |
| | Glycerol | 100 to 200 mg |
| | Distilled water | 100 to 200 mg |
| | Saccharin | 0 to 2 mg |
| | Methyl Paraben | 1 to 2 mg |
| | Polyvinylpyrrolidone | 0 to 2 mg |
| | | |

| 3. | Injectable solution | |
|---|---|---|
| | Compound of 1^{st} embodiment | 0.01 to 20 mg, generally 0.1 to 10mg |
| | Sodium chloride | 8.5 mg |
| | Potassium chloride | 3 mg |
| | Calcium chloride | 4.8 mg |
| | Water for injection, q.s. to | 10 ml |
| | | |

| 4. | Elixir | |
|---|---|---|
| | Compound of 1^{st} embodiment | 0.01 to 20 mg, generally 0.1 to 10mg |
| | Sucrose | 100 mg |
| | Glycerol | 2ml |
| | Carboxymethylcellulose | 20mg |
| | Cherry flavour | 2 mg |
| | Water | q.s. to 10 ml |

### EXAMPLES

The following Examples describe the preparation of compounds according to the invention and are intended to illustrate the invention. The Examples are not be construed as limiting in any way the scope of the present invention. Starting materials for the syntheses described in the following Examples are described in International Patent Application No. PCT/AU93/00103. In these Examples, melting points were taken on a hot stage apparatus and are uncorrected. Proton and phosphorus NMR spectra were recorded at 100 MHz or 300MHz on Perkin Elmer R32 or Broker EM 300 spectrometers, respectively, in CDCl₃ unless otherwise stated. Chemical shifts for proton NMR are ppm downfield from tetramethylsilane; chemical shifts for P³¹ NMR are ppm downfield from 1,2-bis(diphenylphosphino)ethane external standard. Thin layer chromotography (TLC) was performed on silica gel 60-F254 plates (Merck). Compounds were visualized by ultraviolet light and/or 2% aqueous potassium permanganate solution. The composition (by volume) of the TLC solvent systems were (A) hexane/ethyl acetate 3:2, and (B) concentrated NH₄OH/isopropanol 1:3.

### Example 1

### 4S,5S-5,6-Dibenzyl-1,2-(cis-1,2-cyclohexane)dimethyl-4-hydroxy-7-oxo-perhydro-1,2,6-triazepine

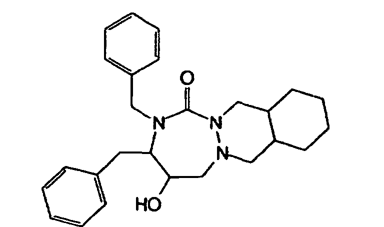

*Step A: 4S,5S-5-benzyl-1,2-(cis-1,2-cyclohexane)dimethyl-4-t-butyldimethylsilyloxy-7-oxo-perhydro-1,2,6-triazepine*: Hydrogen chloride gas was bubbled through the solution of 0.51 g (1.26 mmol) of cis-1,6-3-t-butoxycarbonyl-4-[(2S,3S)-2-hydroxy-3-amino-4-phenylbutyl]-3,4-diazabicyclo[4.4.0]decane (isomer having R_{f}(A) = 0.16 when eluted with 8% methanol in dichloromethane) in 10 ml of 1% solution of methanol in methylene chloride for 30 min at room temperature. After purging the excess of hydrogen chloride, with nitrogen gas the solvent was removed under reduced pressure to give 0.42 g (100% yield) of the hydrochloride salt of cis-1,6-4-[(2S,3S)-3-amino-2-hydroxy-4-phenylbutyl]-3,4-diaza-bicyclo[4.4.0]decane as a hygroscopic, white solid. This was dissolved in 1 ml of dry DMF and 0.114 g (1.68 mmol) of imidazole and 0.21 g (1.38 mmol) of t-butyldimethylsilyl chloride were added under nitrogen. The resulting mixture was stirred overnight at room temperature and evaporated to dryness *in vacuo*. The residue was diluted to 20 ml with ethyl acetate, washed with saturated sodium bicarbonate and dried over anhydrous potassium carbonate and filtered off. The filtrate was evaporated to dryness under reduced pressure and the residue was dissolved in 20 ml of dry dioxane. To this, 0.204 g (1.26 mmol) of 1,1'-carbonyldiimidazole was added and the resulting mixture was stirred for 24 hrs at room temperature. After evaporation of the solvent under reduced pressure the residue was diluted to 15 ml with ethyl acetate and washed with water (3x) and saturated aqueous sodium chloride solution, and then dried over anhydrous magnesium sulfate. Evaporation of the solvent under reduced pressure and purification of the residue by column chromatography (silica gel; hexane/ethyl acetate 3:2) gave 0.095 g (17% yield) of the title compound, melting at 145 - 146°C; R_{f} (A) = 0.43; NMR 0.07, 0.09 (s,s 6H, CH₃); 0.94 (s, 9H, t-butyl CH₃); 1.2 - 2.0 (m, 10H, cyclohexane CH₂, CH); 2.5 - 2.8 (m, 4H, CH₂-3, benzyl CH₂); 3.2 - 3.7 (m, 4H, dimethyl CH₂); 3.9 - 4.0 (m, 3H, CH-4,5, NH); 7.1 - 7.32 (m, 5H, aromatic).
*Step B: 4S,5S-5,6-dibenzyl-1,2-(cis-1,2-cyclohexane)dimethyl-4-t-butyldimethylsilyloxy-7-oxo-perhydro-1,2,6-triazepine*: 4.5 mg (0.15 mmol) of 80% dispersion of sodium hydride in mineral oil was added to a solution of 0.0665 g (0.15 mmol) of the product of Step A in 0.2 ml of dry DMF at room temperature. After stirring for 30 min at room temperature, 0.0179 ml (0.15 mmol) of benzyl bromide was then added. The resulting mixture was stirred overnight, then diluted to 15 ml with ethyl acetate and washed with water, saturated aqueous sodium chloride solution and dried over anhydrous magnesium sulfate. Evaporation of the solvent under reduced pressure and purification of the residue by column chromatography gave 0.029 g (36% yield) of the title compound as a heavy syrup; R_{f}(A) = 0.77; NMR -0.35, - 0.18 (s, s, CH₃); 0.8 (s, 9H, t-butyl CH₃); 1.2 - 2.2 (m, 10H, cyclohexane CH₂, CH); 2.56 - 4.18 (m, 12H, benzyl CH₂, dimethyl CH₂, CH₂-3, CH-4,5); 6.8 - 7.4 (m, 10H, aromatic).
*Step C: 4S,5S-5,6-Dibenzyl-1,2-(cis-1,2-cyclohexane)dimethyl-4-hydroxy-7-oxo-perhydro-1,2,6-triazepine*: A mixture of 29 mg (0.0543 mmol) of the product of Step B and 0.0426 g (0.163 mmol) of tetrabutylammonium fluoride hydrate in 1 ml of anhydrous acetonitrile was stirred at 45±5°C for 3 hrs and evaporated to dryness. The residue was purified by column chromatography (silica gel, hexane/ethyl acetate 3:2) to give 0.019 g (86% yield) of the title compound as a colourless foam; R_{f} (A) = 0.26; NMR 1.2 - 2.1 (m, 18H, cyclohexane CH₂, CH, OH, 3.5 x H₂O); 2.6 - 4.0 (m, 11H, benzyl CH₂, dimethyl CH₂, CH₂-3, CH-5); 4.83 (m, 1H, CH-4); 7.0 - 7.4 (m, 10H, aromatic).

### Example 2

### 4S,5S-1,5,6,Tribenzyl-2-isopropyl-4-hydroxy-7-oxo-perhydro-1,2,6-triazepine

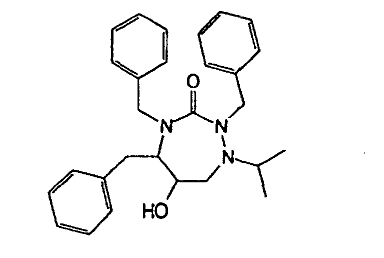

*Step A: 4S,5S-5-benzyl-2-isopropyl-4-t-butyldimethylsilyloxy-7-oxo-perhydro-1,2,6-triazepine*: When t-butyl 3-isopropyl-3-[(2S,3S)-3-amino-2-hydroxy-4-phenylbutyl]carbazate was substituted for cis-1,6-3-t-butoxycarbonyl-4-[(2S,3S)-2-hydroxy-3-amino-4-phenylbutyl]-3,4-diazabicyclo[4.4.0]decane in Step A of Example 1 the identical process afforded the title compound in 20% overall yield; melting point = 131 - 132°C (hexane); R_{f} (A) = 0.18; NMR 0.10, 0.11 (s, s, 6H, silyl CH₃); 0.95 (s, 9H, t-butyl CH₃); 1.1 - 1.35 (m, 6H, isopropyl CH₃); 2.8 - 3.2 (m, 5H, CH₂-3, CH-5, benzyl CH₂); 3.45 (m, 1H, isopropyl CH); 4.18 (m, 1H, CH-4); 4.41 (m, 1H NH-6); 5.63 (s, 1H, NH-1); 7.1 - 7.4 (m, 5H, aromatic).
*Step B: 4S,5S-1,5,6-tribenzyl-2-isopropyl-4-t-butyldimethylsilyloxy-7-oxo-perhydra-1,2,6-triazepine:* A mixture of 0.07 g (0.185 mmol) of the product of Step A and 0.012 g (0.371 mmol) of sodium hydride in 0.2 ml of dry DMF was stirred for 30 min at room temperature, then 0.0441 ml (0.371 mmol) of benzyl bromide was added. The resulting mixture was stirred overnight and worked up as described in Step B of Example 1. The purification of the crude product by column chromatography (silica gel, hexane/ethyl acetate 3:2) gave 0.031 g (30% yield) of the title compound as a colourless syrup; Rf (A) = 0.74, NMR -0.28, - 0.22 (s,s, 6H, silyl CH₃); 0.8 (s, 9H, t-butyl CH₃); 1.0 - 1.35 (m, 6H, isopropyl CH₃); 2.35 - 3.3 (m, 5H, CH₂₋₃, CH-5, 5-benzyl CH₂); 3.45 - 3.82 (m, 2H, isopropyl CH, CH-4); 4.0 - 5.38 (m, 4H, 1,6-benzyl CH₂); 6.6 - 7.8 (m, 15H, aromatic).
   Also, the fractions with Rf (A) = 0.63 were combined and evaporated to dryness under reduced pressure to give 0.061 g (70% yield) of 4S,5S-5,6-dibenzyl-2-isopr-opyl-4-t-butyl-dimethylsilyloxy-7-oxo-perhydro-1,2,6-triazepine as a colourless solid; NMR 0.11 (d, 6H, silyl CH₃); 0.93 (s, 9H, t-butyl CH₃); 1.24 (m, 6H, isopropyl CH₃); 2.4 - 3.4 (m, 5H, CH₂-3, CH-5, 5-benzyl CH₂); 3.75 (m, 1H, isopropyl CH); 4.0 - 4.7 (m, 3H, CH-4, 6-benzyl CH₂); 5.05 (m, 1H, NH); 7.0 - 7.7 (m, 15H, aromatic).
*Step C: 4S,5S-1,5,6-Tribenzyl-2-isopropyl-4-hydroxy-7-oxo-perhydro-1,2,6-triazepine*: When the title compound of Step B was substituted for 4S,5S-5,6-dibenzyl-1,2-(cis-1,2-cyclohexane)-dimethyl-4-t-butyldimethylsilyloxy-7-oxo-perhydro-1,2,6-triazepine in Step C of Example 1, the identical process afforded the title compound with 98% yield as a foam; Rf (A) = 0.68; NMR (CDCl₃) 1.07, 1.19 (d, d, 6H, isopropyl CH₃); 1.58 (s, 1H, OH); 2.6-3.15 (m, 5H, CH₂-3, CH-5, 5-benzyl CH₂); 3.2 - 5.3 (m, 6H, isopropyl CH, CH-4, 1,6-benzyl CH₂); 6.8 - 7.6 (m, 15H, aromatic).

### Example 3

### 4S,5S-5,6-dibenzyl-2-isopropyl-4-hydroxy-7-oxo-perhydro-1,2,6-triazepine

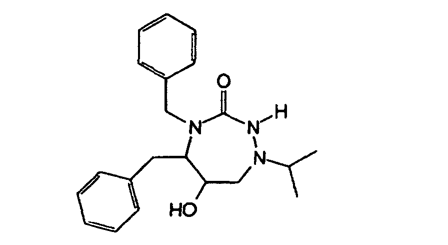

When 4S,5S-5,6-dibenzyl-2-isopropyl-4-t-butyldimethylsilyloxy-7-oxo-perhydro-1,2,6-triazepine was substituted for 4S,5S-1,5,6-tribenzyl-2-isopropyl-4-t-butyldimethylsilyloxy-7-oxo-perhydro-1,2,6-triazepine in Step C of Example 2 the identical process afforded the title compound in 88% yield; melting point = 191 - 193°C; Rf (A) = 0.17; NMR (DMSO-d₆, 80°C) 2.5 - 3.0 (m, 4H, CH₂-3, 5-benzyl CH₂); 3.28 (m, 1H, CH-5); 3.6 (m, 1H, CH-4); 3.8 (m, 1H, isopropyl CH); 4.2 - 4.7 (m, 3H, 6-benzyl CH₂; OH); 5.41 (m, 1H, NH); 7.0 - 7.4 (m, 10H, aromatic).

### Example 4

### t-Butyl 3-isopropyl-3-[(2S, 3S)-2-phosphitoox-y-3-(N-quinaldoyl-L-asparaginyl)amino-4-phenylbutyl carbazate

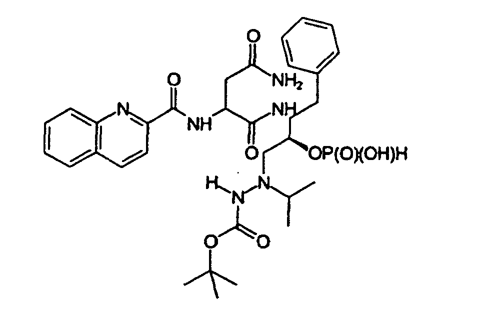

To a mixture of 0.4 g (0.67 mmol) of t-butyl 3-isopropyl-3-[(2S, 3S)-2-hydroxy-3-(N-quinaldoyl-L-asparaginyl)amino-4-phenylbutyl carbazate and 0.12 g (1.47 mmol) of anhydrous phosphorous acid in 1.5 ml of anhydrous pyridine was added 0.28 g (1.4 mmol) of dicyclohexylcarbodiimide at room temperature under nitrogen, with stirring. After stirring for 2 hours at 60°C, the solvent was evaporated under reduced pressure and the residue was treated with 28 ml of 0.1 ml aqueous sodium bicarbonate and vigorously stirred for 1 hour at room temperature. The precipitate was filtered off and washed with water and the filtrate was acidified to pH∼1.5 with concentrated hydrochloric acid. The product was taken up by extraction with ethyl acetate (3 x 50 ml), and the organic phase was dried over anhydrous magnesium sulfate. Evaporation of the solvent gave 0.42 g (95% yield) of the title product as a colourless solid; R_{f} (B) = 0.62; H¹NMR (CDCl₃): 1.08 (m, 6H, isopropyl CH₃); 1.41 (s, 9H, t-butyl CH₃); 2.7 - 4.8 (m, 14H, asn CH₂, butyl CH₂-1, 4; CH-2,3; isopropyl CH; P-OH x 2H₂O); 5.12 (m, 1H, asn CH); 5.89 (s, 0.5 H, PH); 6.2 - 8.5 (m, 15.5 H, aromatic, amide NH, 0.5 PH); 9.02 (m, 1H, asn NH); P³¹NMR (CDCl₃) 14.99 (J_{P-H} = 636 Hz).

### Example 5

### t-Butyl 3-isopropyl-3-[(2S, 3S)-2-phosphonooxy-3-(N-quinaldoyl-L-asparaginyl)amino-4-phenylbutyl carbazate

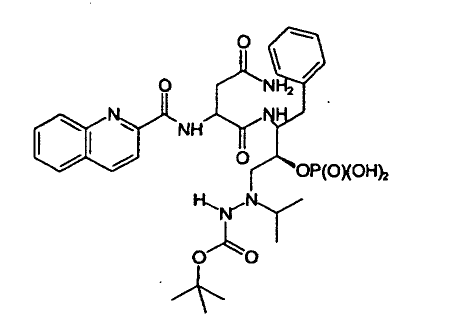

A suspension of 0.4 g (0.6 mmol) of the product of Example 4 in 2 ml of hexamethyldisilazane was stirred for 45 min at 120 ± 5°C. At this point the reaction mixture became homogeneous. To this 0.3 ml of bis(trimethylsityl)peroxide (Cookson, P.G et al., *J. Organometal. Chem*., 1975, 99, C31) was added and stirring was continued for 1 hour at the above temperature. The reaction mixture was cooled to room temperature, then evaporated to dryness *in vacuo.* The residue was dissolved in 20 ml of methanol, evaporated to dryness under reduced pressure and redissolved in 12 ml of 0.1 ml aqueous sodium bicarbonate. The resulting mixture was acidified to pH∼1.5 with concentrated hydrochloric acid, saturated with sodium chloride and extracted with ethyl acetate (3 x 50 ml). The combined organic phase was dried over anhydrous magnesium sulfate and evaporated to dryness to give 0.39 g (96% yield) of the title compound as a colourless solid; R_{f}(B) = 0.07; H¹NMR (CDCl₃): 1.2 (m, 6H, isopropyl CH₃); 1.4 (s, 9H, t-butyl CH₃); 2.8 - 4.2 (m, 8H, asn CH₂ butyl CH₂-1,4, CH-3, isopropyl CH); 4.2 - 6.4 (m, 5H, asn CH, butyl CH-2, NH, POH); 6.5 - 8.4 (m, 14H, aromatic, NH); 8.78 (m, 2H, NH); P³¹NMR (CDCl₃) 9.6 (s).

### Example 6

### cis-1,6-3-t-Butoxycarbonyl-4-[(2S, 3S)-2-phosphitooxy-3-(N-quinaldoyl-L-asparaginyl)amino-4-phenylbutyl]-3,4-diaza-bicyclo[4.4.0]decane

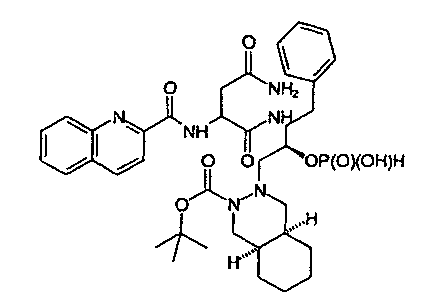

When cis-1,6-3-t-butoxycarbonyl-4-[(2S, 3S)-2-hydroxy-3-(N-quinaldoyl-L-asparaginyl)amino-4-phenylbutyl]-3,4-diaza-hicyclo[4.4.0]decane is substituted for t-butyl 3-isopropyl-3-[(25, 3S)-2-hydroxy-3-(N-quinaldoyl-L-asparaginyl)amino-4-phenylbutyl-carbazate in Example 4, the identical process affords the title compound in 89% yield, as a colourless solid; R_{f}(B) = 0.64; H¹NMR (CDCl₃): 1.1 - 1.8 (m, 19H, t-butyl CH₃, decane CH₂-7,8,9,10, CH-1,6); 2.12 (m, 1H, butyl CH-3); 2.6 - 5.1 (m, 19H, asn CH₂, CH, butyl CH₂-1,4, CH-2, decane CH₂-2,5, POH x 2.5 H₂O); 6.1 - 8.4 (m, 15H, amide NH, PH, aromatic); 9.08 (m, 1H, asn NH); P³¹NMR (CDCl₃) 16.43 (J_{PH} = 700 Hz).

### Example 7

### cis-1,6-3-t-Butoxycarbonyl-4-[(2S, 3S)-2-phosphonooxy-3-(N-quinaldoyl-L-asparaginyl)amino-4-phenylbutyl]-3,4-diaza-bicyclo[4.4.0]decane

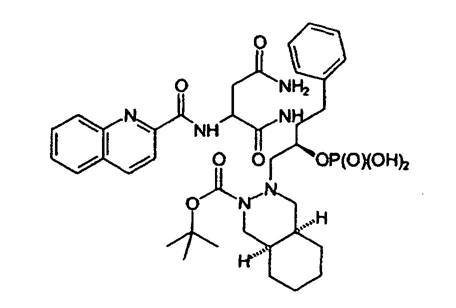

When the product of Example 6 is substituted for t-butyl 3-isopropyl-3-[(2S, 3S)-2-phosphitooxy-3-(N-quinaldoyl-L-asparaginyl)amino-4-phenylbutyl carbazate in Example 5, the identical process affords the title compound in 83% yield, as a colourless solid; R_{f}(B) = 0.12; H¹NMR (CDCl₃): 1.1 - 2.4 (m, 20H, t-butyl CH₃, decane CH₂-7,8,9,10, CH-1,6, butyl CH-3); 2.7 - 3.9 (m, 9H, asn CH₂, butyl CH₂-1,4, CH-2, decane CH₂-5); 5.1 (m, 1H, asn CH); 6.1 - 8.3 (m, 21H, amide NH, aromatic, POH x 2.5 H₂O); 9.05 (m, 1H, asn NH); P³¹NMR (CDCl₃) 10.5 (s).

### Example 8

### In Vivo Removal of Phosphono Group

**Solutions:** The product of Example 5 was converted quantitatively into the corresponding disodium salt by treatment of the free acid with 2 equiv. of 0.2 M sodium bicarbonate and lyophilization of the resulting solution. The stock solutions of the disodium salt of the product of Example 5, for blood and animal experiments, were prepared in sterile water.

**Analyses:** Reverse phase analyses (HPLC) were performed on Waters ternary gradient liquid chromatograph equipped with 996 diode array detector set at 238 nm. Separations were achieved on Alltima RP-18 (250 x 4.6 mm, i.d., 5 µ particles), with the flow rate of 1 ml/min. The isocratic mobile phase composition used for analyses consisted of 40% of 0.1% aqueous trifluoroacetic acid (TFA) and 60% of acetonitrile containing 0.1% TFA and 10% water. The retention time of the product of Example 5 (referred to below as "Prodrug") was in the range of 3.6 - 3.9 minutes and the retention time of t-butyl 3-isopropyl-3-[(2S, 3S)-2-hydroxy-3-(N-quinaldoyl-L-asparaginyl)amino-4-phenylbutyl -carbazate (referred to below as "Drug) was about 6.2 minutes. Detector response was linear from 0.5 to 120 µM for Prodrug and 0.05 to 50 µM for Drug.

**Standards and Sample Processing:** The standards were prepared by serial dilution of Prodrug or Drug in rabbit blood collected into heparinised tubes. Blood samples were transferred into vials containing 150 units of heparin and stored on ice until processed. The blood samples were then separated by centrifuging at 6000 rpm for 10 min. The plasma samples were frozen and stored at -20°C until they were analysed.

**Plasma preparation for HPLC analysis:** An equal volume (100 µL) of thawed plasma and acetonitrile was stirred with a vortex mixer and allowed to stand at room temperature for 5 minutes, then centrifuged at 14000 rpm for 10 minutes. Samples of the supemantant (50 µL) were injected into the chromatograph.

**Transformation of Prodrug into Drug by Blood** was established by measurement of prodrug and drug concentrations in plasma following the prodrug incubation in whole rabbit's blood (100 µM) at 36°C for 19 hours. Fig. 1 shows the concentrations of prodrug and drug under these conditions over 19 hours.

**Transformation of Prodrug into Drug after intravenous (IV) administration** of prodrug (9.2 mg/kg) to rabbit was established by measurement of prodrug/drug concentrations in plasma over 120 min. The formulated product, containing 30 mg/ml of prodrug, was well tolerated by the rabbit. The plasma profiles of prodrug and drug disappearance are shown in Fig. 2.

**Transformation of Prodrug into Drug after Intramuscular (IM) administration** of prodrug (7.9 mg/kg) to rabbit was established by measurement of drug concentrations in plasma over 330 min. The formulated product, containing 30 mg/ml of prodrug was well tolerated by the rabbit. The time dependence of the plasma concentration of the drug are shown in Fig. 3.

When prodrug was administered to a dog orally at a dose of 20mg/kg, the blood plasma concentration of drug was found to be 0.044, 0.141, 0.189, 0.172, 0.164, 0.132, 0.089 and 0.060 µM, respectively, after 5, 15, 30, 47, 63, 93, 124 and 155 minutes. When prodrug was administered to a second dog orally at a dose of 10mg/kg, the blood plasma concentration of drug was found to be 0.137, 0.371, 0.297, 0.242, 0.176, 0.11, 0.071, and 0.050 µM, respectively, after 5, 15, 30, 45, 60, 94, 123 and 154 minutes.

## Claims

1. A compound selected from the group consisting of:
cis- 1,6-3 -t-Butoxycarbonyl-4-[(2 S, 3 S)-2-phosphonooxy-3 -(N-quinaldoyl-L-asparaginyl)amino-4-phenylbutyl] -3 ,4-diaza-bicyclo [4.4.0] decane;
cis- 1,6-3-t-Butoxycarbonyl-4-[(2S, 3 S)-2-phosphitooxy-3 -(N-quinaldoyl-L-asparaginyl)amino-4-phenylbutyl] -3 ,4-diaza-bicyclo [4.4.0] decane;
t-Butyl 3-isopropyl-3-[(2 S, 3 S)-2-phosphonooxy-3 -(N-quinaldoyl-L-asparaginyl)amino-4-phenylbutylcarbazate; and
t-Butyl 3-isopropyl-3 -[(2S, 3 S)-2-phosphitooxy-3-(N-quinaldoyl-L-asparaginyl)amino-4-phenylbutylcarbazate.

2. The compound t-Butyl 3-isopropyl-3-[(2S, 3S)-2-phosphonooxy-3-(N-quinaldoyl-L-asparaginyl)amino-4-phenylbutylcarbazate.

3. A pharmaceutical composition comprising an effective amount of a compound according to Claim 1 together with at least one pharmaceutically acceptable carrier, diluent, excipient and/or adjuvant.

4. A composition according to Claim 3 said composition being for oral administration.

5. The use of a compound according to Claim 1 for the manufacture of a medicament for inhibiting a retroviral protease in a mammal in need of such retroviral protease inhibition.

6. A use according to Claim 5 wherein the retroviral protease is an HIV protease.

7. A use according to Claim 5 or 6 wherein the medicament is for oral administration.

8. The use of a compound according to Claim 2 for the manufacture of a medicament for inhibiting a retroviral protease in a mammal in need of such retroviral protease

9. A use according to Claim 8 wherein the retroviral protease is an HIV protease.

10. The use of a compound according to Claim 1 or 2 for the manufacture of a medicament for the treatment or prophylaxis of acquired immune deficiency syndrome in a mammal in need of such treatment.

11. A pharmaceutical composition comprising an effective amount of a compound according to Claim 2 together with at least one pharmaceutically acceptable carrier, diluent, excipient and/or adjuvant.

12. A composition according to Claim 11 said composition being for oral administration.

## Patentansprüche

1. Verbindung, die aus der Gruppe ausgewählt ist, bestehend aus:
cis-1,6,3-t-Butoxycarbonyl-4-[(2S, 3S)-2-phosphonooxy-3-(N-chinaldoyl-L-asparaginyl)amino-4-phenylbutyl]-3,4-diaza-bicyclo[4.4.0]-decan;
cis-1,6,3-t-Butoxycarbonyl-4-[(2S, 3S)-2-phosphitooxy-3-(N-chinaldoyl-L-asparaginyl)amino-4-phenylbutyl]-3,4-diaza-bicyclo[4.4.0]-decan;
t-Butyl-3-isopropyl-3-[(2S, 3S)-2-phosphonooxy-3-(N-chinaldoyl-L-asparaginyl)amino-4-phenylbutylcarbazat; und
*t*-Butyl-3-isopropyl-3-[(2S, 3S)-2-phosphitooxy-3-(N-chinaldoyl-L-asparaginyl)amino-4-phenylbutylcarbazat.

2. Verbindung t-Butyl-3-isopropyl-3-[(2S, 3S)-2-phosphonooxy-3-(N-chinaldoyl-L-asparaginyl)amino-4-phenylbutylcarbazat.

3. Pharmazeutische Zusammensetzung umfassend eine wirksame Menge einer Verbindung nach Anspruch 1 zusammen mit mindestens einem pharmazeutisch verträglichen Träger, Verdünnungsmittel, Hilfsstoff und/oder Adjuvans.

4. Zusammensetzung nach Anspruch 3, wobei die Zusammensetzung zur oralen Verabreichung vorgesehen ist.

5. Verwendung einer Verbindung nach Anspruch 1 zur Herstellung eines Medikamentes zur Inhibition einer retroviralen Protease bei einem Säuger mit Bedarf an einer solchen retroviralen Protease-Inhibition.

6. Verwendung nach Anspruch 5, worin die retrovirale Protease eine HIV-Protease darstellt.

7. Verwendung nach Anspruch 5 oder 6, worin das Medikament zur oralen Verabreichung vorgesehen ist

8. Verwendung einer Verbindung nach Anspruch 2 zur Herstellung eines Medikamentes zur Inhibition einer retroviralen Protease bei einem Säuger mit Bedarf an einer solchen retroviralen Protease-Inhibition.

9. Verwendung nach Anspruch 8 worin die retrovirale Protease eine HIV-Protease darstellt.

10. Verwendung einer Verbindung nach Anspruch 1 oder 2 zur Herstellung eines Medikamentes zur Behandlung oder Prophylaxe des Acquired Immune Deficiency Syndrome bei einem Säuger mit Bedarf an einer solchen Behandlung.

11. Pharmazeutische Zusammensetzung umfassend eine wirksame Menge einer Verbindung nach Anspruch 2 zusammen mit mindestens einem pharmazeutisch verträglichen Träger, Verdünnungsmittel, Hilfsstoff und/oder Adjuvans.

12. Zusammensetzung nach Anspruch 11, wobei die Zusammensetzung zur oralen Verabreichung vorgesehen ist.

## Revendications

1. Un composé choisi dans le groupe constitué de:
cis-1,6-3-t-butoxycarbonyl-4-[(2S, 3S)-2-phosphonooxy-3-(N-quinaldoyl-L-asparaginyl)amino-4-phénylbutyl]-3,4-diaza-bicyclo[4.4.0]décane;
cis-1,6-3-t-butoxycarbonyl-4-[(2S, 3S)-2-phosphitooxy-3-(N-quinaldoyl-L-asparaginyl)amino-4-phénylbutyl]-3,4-diaza-bicyclo[4.4.0]décane
3-isopropyl-3-[(2S, 3S)-2-phosphonooxy-3-(N-quinaldoyl-L-asparaginyl)-amino-9-phénylbutylcarbazate de t-butyle; et
3-isopropyl-3-[(2S, 3S)-2-phosphitooxy3-(N-quinaldoyl-L-asparaginyl)amino-4-phénylbutylcarbazate de t-butyle.

2. Le composé 3-isopropyl-3-[(2S, 3S)-2-phosphonooxy-3-(N-quinaldoyl-L-asparaginyl)amino-4-phénylbutylcarbazate de t-butyle.

3. Une composition pharmaceutique comprenant une quantité efficace d'un composé selon la Revendication 1 ainsi qu'au moins un véhicule phazmaceutiquement acceptable, diluant, excipient et/ou adjuvant.

4. Une composition selon la Revendication 3, ladite composition étant pour l'administration orale.

5. L'utilisation d'un composé selon la Revendication 1 pour la fabrication d'un médicament pour inhiber une protéase rétrovirale chez un mammifère ayant besoin d'une telle inhibition de protéase rétrovirale.

6. Une utilisation selon la Revendication 5, dans laquelle la protéase rétrovirale est une protéase de VIH.

7. Une utilisation selon la Revendication 5 ou 6, dans laquelle le médicament est pour l'administration orale.

8. L'utilisation d'un composé selon la Revendication 2 pour la fabrication d'un médicament pour inhiber une protéase rétrovirale chez un mammifère ayant besoin d'une telle inhibition de protéase virale.

9. Une utilisation selon la Revendication 8, dans laquelle la protéase rétrovirale est une protéase de VIH.

10. L'utilisation d'un composé selon la Revendication 1 ou 2 pour la fabrication d'un médicament pour le traitement ou la prophylaxie du syndrome d'immunodéficience acquise chez un mammifère ayant besoin d'un tel traitement.

11. Une composition pharmaceutique comprenant une quantité efficace d'un composé selon la Revendication 2 ainsi qu'au moins un véhicule pharmaceutiquement acceptable, diluant, excipient et/ou adjuvant.

12. Une composition selon la Revendication 11, ladite composition étant pour l'administration orale.
